# EUROPEAN PATENT SPECIFICATION

(11) **EP 2 208 734 B1**
(45) Date of publication and mention of the grant of the patent: **25.01.2012**
(21) Application number: 08844720.6
(22) Date of filing: 28.10.2008
(51) Int. Cl.: C07K 14/47, A23K 1/16, A23L 1/305, A61K 38/00, A61P 19/08, C07K 2/00

(54) **FOOD MATERIAL FOR INHIBITING THE FORMATION OF OSTEOCLAST**
NAHRUNGSMITTEL ZUR INHIBIERUNG DER OSTEOCLASTBILDUNG
MATIÈRE D'ALIMENT POUR INHIBER LA CRÉATION D'OSTÉOCLASTE

(30) Priority: 01.11.2007 JP 2007285423
(43) Date of publication of application: 21.07.2010
(73) Proprietor: Megmilk Snow Brand Co., Ltd., Sapporo-shi Hokkaido 0650043 (JP)
(72) Inventor: SERIZAWA, Atsushi, Kawagoe-shi Saitama 350-1165 (JP); MORITA, Yoshikazu, Kawagoe-shi Saitama 350-1165 (JP); UETSUJI, Daisuke, Kawagoe-shi Saitama 350-1165 (JP); ONO, Aiko, Kawagoe-shi Saitama 350-1165 (JP); MATSUYAMA, Hiroaki, Kawagoe-shi Saitama 350-1165 (JP); HIGURASHI, Satoshi, Kawagoe-shi Saitama 350-1165 (JP)
(74) Representative: Wakerley, Helen Rachael
(86) International application number: PCT/JP2008/003071
(87) International publication number: WO 2009/057287

(56) References cited:
- EP-A2- 0 704 218
- EP-A2- 1 161 881
- JP-A- 8 151 331
- JP-A- 10 007 585
- JP-A- 2001 346 519
- JP-A- 2004 115 509
- JP-A- 2004 115 509
- JP-A- 2005 060 321
- US-A1- 2004 038 265
- US-B2- 7 247 331

## Description

### TECHNICAL FIELD

The present invention relates to a milk protein fraction or a milk protein fraction degradation product that exhibits an osteoclastogenesis inhibitory effect.
Since the milk protein fraction or the milk protein fraction degradation product according to the present invention exhibits a osteoclastogenesis inhibitory effect, the milk protein fraction or the milk protein fraction degradation product is useful as an osteoclastogenesis inhibitory agent that aims at preventing or treating bone diseases or strengthening a bone, and is also useful as an active ingredient of a pharmaceutical, food, drink, or feed that aims at preventing or treating bone diseases or inhibiting osteoclastogenesis.

### BACKGROUND ART

In recent years, various bone diseases, such as osteoporosis, bone fractures, lumbago or the like have increased along with the progressive increase in the elderly population. In a bone tissue, osteogenesis and bone resorption incessantly occur. In a young person, a balance between osteogenesis and bone resorption is kept, but the balance is disrupted to bone resorption owing to various causes with aging (uncoupling). Continuance of this state for a long period of time makes the bone tissue fragile, resulting in occurrence of various bone diseases, such as osteoporosis, bone fractures, and lumbago. It is considered that prevention of the uncoupling enables prevention of various bone diseases, such as osteoporosis, bone fractures, and lumbago.

Conventionally, in order to prevent the uncoupling to prevent or treat bone diseases, the following methods have been performed: (1) calcium supplementation by diet, (2) light exercise, (3) insolation, (4) medication, and the like. For calcium supplementation by diet, there are used calcium salts, such as calcium carbonate, calcium phosphate or the like, or natural calcium agents, such as eggshell, fish bone powder or the like. However, these materials are not necessarily suitable for oral intake. Jogging, walking, or the like may be recommended as light exercise. However, even light exercise is troublesome for a person whose body has weakened, and it is almost impossible for a bedridden old person to do exercise. It is considered that insolation is a good means to supplement activated vitamin D₃, but it is not sufficient in itself. 1α-Hydroxyvitamin D₃, a calcitonin preparation, or the like is used for administration of a pharmaceutical, and is known to be effective for treating osteoporosis. However, these substances are pharmaceuticals themselves and cannot be used as a food material.

The inventors of the present invention have searched for a bone-strengthening factor contained in milk in order to obtain a bone-strengthening substance that can be used as a food material. As a result, the inventors found that a protein and a peptide mixture obtained by removing a salt derived from a milk serum from a water-soluble fraction of a milk serum protein exhibit a bone-strengthening effect (see Patent Document 1, for example). The inventors found that a fraction obtained by subjecting an aqueous solution of the protein and the peptide mixture to an ethanol treatment, a heat treatment, a salting treatment, and an ultrafiltration membrane treatment exhibits an osteoblast growth promoting effect and a bone-strengthening effect (see Patent Documents 2 and 3, for example). The inventors further found that a basic protein contained in milk exhibits an osteoblast growth promoting effect, a bone-strengthening effect, and a bone resorption prevention effect (see Patent Document 4, for example).
Patent Document 1: Japanese Patent No. 3160862
Patent Document 2: Japanese Patent No. 3092874
Patent Document 3: JP-A-H05-320066
Patent Document 4: Japanese Patent No. 3112637

### DISCLOSURE OF THE INVENTION

### PROBLEMS TO BE SOLVED BY THE INVENTION

An object of the present invention is to provide a milk protein fraction or a milk protein fraction degradation product that exhibits an osteoclastogenesis inhibitory effect and can be used as a food material, an osteoclastogenesis inhibitory agent containing the milk protein fraction or the milk protein fraction degradation product that exhibits an osteoclastogenesis inhibitory effect, and a pharmaceutical, food, drink, or feed for inhibiting osteoclastogenesis containing the milk protein fraction or the milk protein fraction degradation product that exhibits an osteoclastogenesis inhibitory effect.

### MEANS FOR SOLVING THE PROBLEMS

The inventors searched for a novel osteoclastogenesis inhibitory material, and found that a fraction exhibiting a high osteoclastogenesis inhibitory effect as compared with a known food material could be obtained. Based on those findings, the inventors thus obtained an osteoclastogenesis inhibitory agent containing the milk protein fraction or the milk protein fraction degradation product that exhibits an osteoclastogenesis inhibitory effect, and a pharmaceutical, food, drink, or feed containing the milk protein fraction or the milk protein fraction degradation product that exhibits an osteoclastogenesis inhibitory effect.

Specifically, the present invention is constituted as follows:
(A) A milk protein fraction having an osteoclastogenesis inhibitory effect and following characteristics;
   (1) the milk protein fraction is derived from milk,
   (2) the milk protein fraction contains proteins having a molecular weight of 80,000 to 85,000 daltons determined by sodium dodecyl sulfate-polyacrylamide gel electrophoresis (SDS-PAGE),
   (3) the milk protein fraction contains 16 to 18 wt% of basic amino acids in the constituent amino acid composition, and has a basic amino acid/acidic amino acid ratio of 0.65 to0.85, and
   (4) the milk protein fraction has an osteoclastogenesis inhibitory effect.
(B) A milk protein fraction degradation product obtained by degrading the above milk protein fraction with a protease.
(C) An osteoclastogenesis inhibitory agent comprising the milk protein fraction or the milk protein fraction degradation product according to (A) or (B), respectively.
(D) A pharmaceutical for inhibiting osteoclastogenesis comprising the milk protein fraction or the milk protein fraction degradation product according to (A) or (B), respectively.
(E) A food or drink for inhibiting osteoclastogenesis comprising the milk protein fraction or the milk protein fraction degradation product according to (A) or (B), respectively.
(F) A feed for inhibiting osteoclastogenesis comprising the milk protein fraction or the milk protein fraction degradation product according to (A) or (B), respectively.

### EFFECTS OF THE INVENTION

The osteoclastogenesis inhibitory agent containing the milk protein fraction or the milk protein fraction degradation product that exhibits an osteoclastogenesis inhibitory effect as an active ingredient, and the osteoclastogenesis inhibitory pharmaceutical, food, drink, or feed containing the milk protein fraction or the milk protein fraction degradation product that exhibits an osteoclastogenesis inhibitory effect according to the present invention inhibit the osteoclastogenesis in a body when taken orally.
Therefore, the osteoclastogenesis inhibitory agent containing the milk protein fraction or the milk protein fraction degradation product as an active ingredient, and the osteoclastogenesis inhibitory pharmaceutical, food, drink, or feed containing the milk protein fraction or the milk protein fraction degradation product that exhibits an osteoclastogenesis inhibitory effect according to the present invention exhibit a bone-strengthening effect by inhibiting the osteoclastogenesis in the body of a human or an animal, and thus are effective for suppressing a decrease in bone mass due to osteoporosis or the like.

### BEST MODE FOR CARRYING OUT THE INVENTION

The present invention relates to an osteoclastogenesis inhibitory agent including a milk protein fraction or a milk protein fraction degradation product that exhibits an osteoclastogenesis inhibitory effect as an active ingredient, as well as an osteoclastogenesis inhibitory pharmaceutical, food or drink, and feed including a milk protein fraction or a milk protein fraction degradation product that exhibits an osteoclastogenesis inhibitory effect.

The milk protein fraction according to the present invention may be obtained, for example, by bringing a milk raw material, such as skim milk, milk serum or the like into contact with a cation-exchange resin, washing the cation-exchange resin with 0.6M sodium chloride solution, and eluting the milk protein adsorbed on the cation-exchange resin using a 1.0 to 1.5M sodium chloride eluant. Note that salt such as a potassium salt, an ammonium salt, a phosphate, an acetate, a carbonate, or the like may be used in addition to sodium chloride. The milk protein fraction according to the present invention may be obtained by appropriately adjusting the ionic strength of the washing agent to 0.55 to 0.65 and the ionic strength of the elution solution to 1.0 or more, preferably 1.0 to 0.2. Furthermore, the milk protein fraction according to the present invention may be obtained by collecting the eluted fraction, desalting or concentrating the fraction using a reverse osmosis (RO) membrane, electrodialysis (ED), or the like, and optionally drying the resulting product. Examples of the reverse osmosis (RO) membrane include Desal-3 (manufactured by Desalination), HR-95 (manufactured by Dow Danmark), NTR-729HF (manufactured by Nitto Denko Corporation), and the like. Examples of an electrodialysis (ED) system include electrodialysis systems manufactured by Yuasa-Ionics Inc. and Nippon Rensui Co., Ltd.

As a method of obtaining a trace protein fraction derived from milk, a method of obtaining a protein fraction by bringing milk or a raw material derived from milk into contact with a cation exchanger, and eluting the basic protein fraction that is adsorbed on the cation exchanger using an eluant that has a pH of more than 5 and an ionic strength of more than 0.5 (JP-A-H05-202098), a method of obtaining a protein fraction using an alginic acid gel (JP-A-S61-246198), a method of obtaining a protein fraction from a milk serum using porous inorganic particles (JP-A-H01-86839), a method of obtaining a protein fraction from milk using a sulfated ester compound (JP-A-S63-255300), and the like have been known. Protein fraction that is derived from milk may be obtained by those methods in the present invention.

The milk protein fraction thus collected may be normally powdered by freeze-drying or the like before use.

The milk protein fraction that exhibits an osteoclastogenesis inhibitory effect used in the present invention preferably contains 16 to 18 wt% of basic amino acids in the constituent amino acid composition, and has a basic amino acid/acidic amino acid ratio of 0.65to0.75. The effect of the present invention may not be achieved if the content of basic amino acids or the basic amino acid/acidic amino acid ratio is outside the above range. The present invention product is a mixture containing proteins having a molecular weight of 80,000 to 85,000 daltons, and the isoelectric point of 7 to 8 as a main component.

The milk protein fraction degradation product has the same amino acid composition as that of the milk protein fraction. For example, a milk protein fraction degradation product having an average molecular weight of 4000 or less may be obtained by treating a milk protein fraction obtained by the above method with a protease such as pepsin, trypsin, chymotrypsin or the like, and optionally treating the resulting product with a protease such as pancreatin or the like. The milk protein fraction degradation product is normally powdered by freeze-drying or the like before use.

As milk or a raw material derived from milk which can be used as source of the milk protein fraction according to the present invention that exhibits an osteoclastogenesis inhibitory effect, cow milk, human milk, goat milk, ewe milk or the like may be given. Such milks may be used as is, or recombined milk, skim milk, whey, or the like derived from such milks may be used.

The milk protein fraction or the milk protein fraction degradation product that exhibits an osteoclastogenesis inhibitory effect and is an active ingredient may be used as is when administering the osteoclastogenesis inhibitory agent according to the present invention. Note that it is also possible to use after being formulated into a powdered pharmaceutical, granules, a tablet, a capsule, a drinkable preparation, or the like in accordance with a conventional method. Moreover, the milk protein fraction or the milk protein fraction degradation product, as is or after formulating a preparation thereof, may be added to a nutrient preparation, food and drink, or the like to achieve an osteoclastogenesis inhibitory effect. Since the milk protein fraction or the milk protein fraction degradation product according to the present invention is relatively stable against heat, the milk protein fraction or the milk protein fraction degradation product can be heat-sterilized under conventional conditions.

In the present invention, in order to achieve an osteoclastogenesis inhibitory effect the dosage or the like may be appropriately determined taking account of weight, sex, age, and the like. The milk protein fraction or the milk protein fraction degradation product may be adjusted the formulating amount thereof so that a normal adult takes the milk protein fraction or the milk protein fraction degradation of the present invention in an amount of 10 to 100 mg/day. That is, the milk protein fraction or the milk protein fraction degradation product according to the present invention is effective at a low dosage. In the present invention, the ingredient having an osteoclastogenesis inhibitory effect exerts the osteoclastogenesis inhibitory effect when orally administered an osteoclastogenesis inhibitory agent or a pharmaceutical, food and drink, or feed formulated the osteoclastogenesis inhibitory agent

The present invention is further described below by way of reference examples, examples, and test examples. Note that the following examples merely illustrate several aspects of the present invention, and should not be construed as limiting the present invention.

### Reference example 1

A milk protein fraction exhibiting an osteoclastogenesis inhibitory effect which was commercially available was prepared in accordance with the following method (see Japanese Patent No. 3112637).
A column (diameter: 10 cm) loaded with 0.5 litters of sulfonated Chitopearl (cation-exchange resin; manufactured by Fuji Spinning Co., Ltd.) was sufficiently washed with deionized water. After passing 50 1 of unsterilized skim milk through the column at a flow rate of 100 ml/min, the column was sufficiently washed with deionized water. 2.5 1 of a 0.05M phosphate buffer (pH 7.0) containing 0.95M sodium chloride was then passed through the column to elute proteins adsorbed on the resin. The eluate was desalted and concentrated by means of a reverse osmosis (RO) membrane treatment, and then freeze-dried to obtain a powdery milk protein fraction. The above procedure was repeated twice to obtain 104 g of a protein fraction. The protein fraction had an isoelectric point of 7.0 to 8.5. The content of basic amino acids in the protein fraction was 17.8%.

### Example 1

A column (diameter: 10 cm) loaded with 0.5 1 of sulfonated Chitopearl (cation-exchange resin; manufactured by Fuji Spinning Co., Ltd.) was sufficiently washed with deionized water. After passing 501 of unsterilized skim milk through the column at a flow rate of 100 ml/min, the column was sufficiently washed with 0.05M phosphate buffer (pH 7.0) containing 0.55M sodium chloride. 2.5 1 of a 0.05M phosphate buffer (pH 7.0) containing 1.45M sodium chloride was then passed through the column to elute proteins adsorbed on the resin. The eluate was desalted and concentrated by means of a reverse osmosis (RO) membrane treatment, and then freeze-dried to obtain a powdery milk protein fraction. The above procedure was repeated two times to obtain 48 g of a protein fraction. The protein fraction had a molecular weight of 80,000 to 85,000 daltons and an isoelectric point of 7.0 to 8.0. The content of basic amino acids in the constituent amino acid contained in the protein fraction was 16 to 18%. The protein fraction had a basic amino acid/acidic amino acid ratio of 0.65 to 0.85.

### Example 2

A column (diameter: 10 cm) loaded with 0.5 1 of sulfonated Chitopearl (cation-exchange resin; manufactured by Fuji Spinning Co., Ltd.) was sufficiently washed with deionized water. After passing 501 of unsterilized skim milk through the column at a flow rate of 100 ml/min, the column was sufficiently washed with a 0.05M phosphate buffer (pH 7.0) containing 0.55M sodium chloride. 2.51 of a 0.05M phosphate buffer (pH 7.0) containing 1.0M sodium chloride was then passed through the column to elute proteins adsorbed on the resin. The eluate was desalted and concentrated by means of a reverse osmosis (RO) membrane treatment, and then freeze-dried to obtain a powdery milk protein fraction. The above procedure was conducted once to obtain 22.1 g of a milk protein fraction. The protein fraction had a molecular weight of 80,000 to 85,000 daltons and an isoelectric point of 7.0 to 8.0. The content of basic amino acids in the constituent amino acid contained in the protein fraction was 16 to 18%. The protein fraction had a basic amino acid/acidic amino acid ratio of 0.65 to 0.85.

### Example 3

A column (diameter: 10 cm) loaded with 0.5 1 of sulfonated Chitopearl (cation-exchange resin; manufactured by Fuji Spinning Co., Ltd.) was sufficiently washed with deionized water. After passing 501 of unsterilized skim milk through the column at a flow rate of 100 ml/min, the column was sufficiently washed with a 0.05M phosphate buffer (pH 7.0) containing 0.65M sodium chloride. 2.5 1 of a 0.05M phosphate buffer (pH 7.0) containing 2.0M sodium chloride was then passed through the column to elute proteins adsorbed on the resin. The eluate was desalted and concentrated by means of a reverse osmosis (RO) membrane treatment, and then freeze-dried to obtain a powdery milk protein fraction. The above procedure was repeated twice to obtain 48.8 g of a milk protein fraction. The protein fraction had a molecular weight of 80,000 to 85,000 daltons and an isoelectric point of 7.0 to 8.0. The content of basic amino acids in the constituent amino acid contained in the protein fraction was 16 to 18%. The protein fraction had a basic amino acid/acidic amino acid ratio of 0.65 to 0.85.

### Example 4

22.1 g of the milk protein fraction obtained in Example 2 was dissolved in 8 l of distilled water. After adding trypsin (manufactured by Kanto Kagaku Co., Ltd.) so as to be the concentration of 2%, the milk protein fraction was hydrolyzed at 37°C for one hour with stirring. After the mixture was neutralized to pH 6.6 with a sodium hydroxide solution, 1% pancreatin (manufactured by Sigma) was added thereto. The mixture was then reacted at 37°C for two hours. After completion of the reaction, the protease was inactivated by heating the mixture at 80°C for 10 minutes to obtain 20.8 g of a milk protein fraction degradation product.

### Example 5

48.8 g of the milk protein fraction obtained in Example 3 was dissolved in 101 of distilled water. After adding pepsin (manufactured by Kanto Kagaku Co., Ltd.) so as to be the concentration of 2%, the milk protein fraction was hydrolyzed at 37°C for one hour with stirring. After the mixture was neutralized to pH 6.8 with a sodium hydroxide solution, 1% pancreatin (manufactured by Sigma) was added thereto. The mixture was then reacted at 37°C for two hours. After completion of the reaction, the protease was inactivated by heating the mixture at 80°C for 10 minutes to obtain 46.7 g of a milk protein fraction degradation product.

### Test example 1

An osteoclastogenesis inhibitory factor (OPG) produced by an osteoblast is known as a differentiation inhibitor of physiological osteoclast. The osteoblast OPG production promotion effect of each of the milk protein fractions obtained in Reference example 1 and Example 1 was determined as the osteoclastogenesis inhibitory effect. Specifically, human osteoblast MG63 were suspended in an MEM-E medium (manufactured by GIBCO, fetal bovine serum: 10%, non-essential amino acid solution (manufactured by GIBCO): 1%, penicillin-streptomycin solution (manufactured by GIBCO): 1%) so that the cell density was 2×10⁴ cells/ml, and added to a 96-well plate (200 µl/well). The cells were cultured for four days in a 5% carbon dioxide incubator. After removing the medium by suction, the milk protein fraction obtained in the above MEM-E medium (160 µl/well), and the MEM-E medium of Example 1 or Reference example 1 in which the milk protein fraction was dissolved to a concentration of 1.0 mg/ml or 0.1 mg/ml, respectively, were added (40 µl/well), and the cells were further cultured for four days. The culture supernatant liquid was collected, and the OPG concentration in the culture supernatant liquid was measured using a human OPG-ELISA kit (manufactured by BIOMEDICA). The cells cultured in only an MEM-E medium to which the sample was not added were used as a control.

### The results are shown in Table 1.

**Table 1**

| Sample | Final concentration | OPG concentration (pmol/L) |
|---|---|---|
| Control | --- | 125 ± 10.9 (%, ± SD) |
| Milk protein fraction (Reference example 1) | 1.0 mg/ml | 162 ± 13.1 |
| Milk protein fraction (Example 1) | 1.0 mg/ml | 368 ± 24.7 |
| Milk protein fraction (Example 1) | 0.1 mg/ml | 182.9 ± 15.6 |

The milk protein fractions obtained in Reference Example 1 and Example 1 both promoted OPG production of the human osteoblast MG63. The OPG production promotion effect of the milk protein fraction obtained in Example 1 was significantly higher than that of the milk protein fraction obtained in Reference Example 1. This indicates that the milk protein fraction obtained in Example 1 exhibits an excellent osteoclastogenesis inhibitory effect.

### Test example 2

Each bone-strengthening effect of the protein fractions obtained in Reference example 1 and Example 1 was investigated by animal experiments. Wistar rats (female, 4 weeks old) were used for the animal experiments. After preliminary feeding for one week, the ovary was removed from each rat. A calcium-deficient food was then fed to the rats for five weeks. The rats from which the ovary was removed and to which the calcium-deficient food were fed for five weeks had osteoporosis symptom obviously. The rats having osteoporosis symptom were divided into 3 experiment groups (seven rats per each group) of a control group (Group A) that was administered no milk protein fraction, a group (Group B) that was administered 0.1 wt% of the milk protein fraction obtained in Reference example 1, and a group (Group C) that was administered 0.1 wt% of the milk protein fraction obtained in Example 2. A test feed shown in Table 2 was fed to each group for four months. The nitrogen content in each test feed was evenly adjusted to 17.06% using casein. Each test feed was blended with 300 mg of calcium, 230 mg of phosphorus and 50 mg of magnesium per 100 g of the test feed.

**Table 2**

| | Group | | |
|---|---|---|---|
| | A | B | C |
| Casein | 20.0 | 19.9 | 19.9 |
| Cornstarch | 15.0 | 15.0 | 15.0 |
| Cellulose | 5.0 | 5.0 | 5.0 |
| Com oil | 5.0 | 5.0 | 5.0 |
| Vitamin mix | 1.0 | 1.0 | 1.0 |
| Mineral mix | 2.65 | 2.65 | 2.65 |
| Sucrose | 51.05 | 51.05 | 51.05 |
| DL-methionine | 0.3 | 0.3 | 0.3 |
| Milk protein fraction obtained in Reference example 1 | - | 0.1 | - |
| Milk protein fraction obtained in Example 1 | - | - | 0.1 |

(wt%)

The thighbone was removed from each rat that was fed for four months, and the bone mineral content was measured by dual energy X-ray absorptiometry (DEXA) using a bone mineral analyzer ("DCS-600" manufactured by Aloka). The thighbone mineral content of the control group was also measured. The results are shown in Table 3. As shown in Table 3, the thighbone mineral content of the rats of the group (group B) that was administered the milk protein fraction obtained in Reference example 1 and the group (group C) that was administered the milk protein fraction obtained in Example 2 was significantly higher than that of the rats of the control group after feeding the test feed for four months. Each milk protein fraction was admitted to exhibit a bone mineral content increase effect, i.e., osteoclastogenesis inhibitory effect. However, the effect achieved by the present invention product was higher than that of the milk protein fraction obtained in Reference Example 1. A similar effect was observed when using the milk protein fraction degradation products obtained in Examples 4 and 5 (but not shown the results).

**Table 3**

| | Bone mineral content (mg ± SD) |
|---|---|
| Control (Group A) | 129.4 ± 4.2 |
| Milk protein fraction obtained in Reference example 1 (Group B) | 135.2 ± 3.4 |
| Milk protein fraction obtained in Example 2 (Group C) | 140.1 ± 2.5 |

### Example 6

100 mg of the milk protein fraction obtained in Example 1 was added with 93.4 g of crystalline glucose hydrate, 5 g of calcium carbonate, 1 g of a sugar ester, and 0.5 g of flavor, and mixed. The resultant was then formed into a tablet to obtain an osteoclastogenesis inhibitory agent according to the present invention.

### Example 7

The components were mixed in accordance with the composition shown in Table 4 to obtain a dough. The dough was formed and baked to produce a cookie for inhibiting osteoclastogenesis.

**Table 4**

| | |
|---|---|
| Flour | 50.0 (wt%) |
| Sugar | 20.0 |
| Salt | 0.5 |
| Margarine | 12.5 |
| Egg | 12.1 |
| Water | 4.0 |
| Sodium hydrogen carbonate | 0.1 |
| Ammonium bicarbonate | 0.2 |
| Calcium carbonate | 0.5 |
| Milk protein fraction powder (Example 1) | 0.1 |

### Example 8

An osteoclastogenesis inhibitory fruit juice drink having a composition shown in Table 5 was produced.

**Table 5**

| | |
|---|---|
| Isomerized sugar mix | 15.0 (wt%) |
| Fruit juice | 10.0 |
| Citric acid | 0.5 |
| Milk protein fraction powder (Example 1) | 0.5 |
| Flavor | 0.1 |
| Calcium | 0.1 |
| Water | 73.8 |

### Example 9

The ingredients were mixed in accordance with the formulation shown in Table 6 to produce a dog food for inhibiting osteoclastogenesis.

**Table 6**

| | |
|---|---|
| Milk protein fraction powder (Example 1) | 2.5 (wt%) |
| Skim milk powder | 13.5 |
| Soybean cake | 12.0 |
| Soybean oil | 4.0 |
| Com oil | 2.0 |
| Palm oil | 27.0 |
| Com starch | 14.0 |
| Flour | 9.0 |
| Bran | 2.0 |
| Vitamin mix | 9.0 |
| Mineral mix | 2.0 |
| Cellulose | 3.0 |

### Example 10

Each ingredient was mixed in accordance with the formulation shown in Table 7, and formed under pressure to produce an osteoclastogenesis inhibitory tablet containing the milk protein fraction degradation product obtained in Example 4.

**Table 7**

| | |
|---|---|
| Crystalline glucose hydrate | 59.4 (wt%) |
| Milk protein fraction degradation product (Example 4) | 16.0 |
| Corn starch | 12.0 |
| Cellulose | 4.0 |
| Com oil | 4.0 |
| Vitamin mix (including choline) | 1.0 |
| Mineral mix | 3.6 |

### Example 11

Each ingredient was mixed in accordance with the formulation shown in Table 8, and emulsified at 85°C to produce an osteoclastogenesis inhibitory processed cheese formulating the milk protein fraction degradation product obtained in Example 5.

**Table 8**

| | |
|---|---|
| Gouda cheese | 43.0 (wt%) |
| Cheddar cheese | 43.0 |
| Sodium citrate | 2.0 |
| Milk protein fraction degradation product (Example 5) | 0.5 |
| Calcium derived from milk | 1.0 |
| Water | 10.5 |

### INDUSTRIAL APPLICABILITY

The milk protein fraction and the milk protein fraction degradation product according to the present invention exhibits an osteoclastogenesis inhibitory effect, and is effective for suppressing a decrease in bone mass observed in osteoporosis. Therefore, the milk protein fraction or the milk protein fraction degradation product may be used for an osteoclastogenesis-inhibiting agent containing the milk protein fraction or the milk protein fraction degradation product that exhibits an osteoclastogenesis inhibitory effect as an active ingredient, and an osteoclastogenesis-inhibiting pharmaceutical, food, drink, or feed containing the milk protein fraction or the milk protein fraction degradation product that exhibits an osteoclastogenesis inhibitory effect.

## Claims

1. A milk protein fraction having following characteristics (1) to (4);
(1) the milk protein fraction is derived from milk,
(2) the milk protein fraction contains proteins having a molecular weight of 80,000 to 85,000 daltons determined by sodium dodecyl sulfate-polyacrylamide gel electrophoresis (SDS-PAGE),
(3) the milk protein fraction contains 16 to 18 wt% of basic amino acids in the constituent amino acid composition, and has a basic amino acid/acidic amino acid ratio of 0.65 to 0.85, and
(4) the milk protein fraction has an osteoclastogenesis inhibitory effect.

2. A milk protein fraction degradation product obtained by degrading the milk protein fraction according to claim 1 with a protease.

3. An osteoclastogenesis inhibitory agent comprising the milk protein fraction according to claim 1 or the milk protein fraction degradation product according to claim 2.

4. A pharmaceutical for use in inhibiting osteoclastogenesis comprising the milk protein fraction according to claim 1 or the milk protein fraction degradation product according to claim 2.

5. A food or drink for use in inhibiting osteoclastogenesis comprising the milk protein fraction according to claim 1 or the milk protein fraction degradation product according to claim 2.

6. A feed for use in inhibiting osteoclastogenesis comprising the milk protein fraction according to claim 1 or the milk protein fraction degradation product according to claim 2.

## Patentansprüche

1. Milchproteinfraktion mit den folgenden Eigenschaften (1) bis (4):
(1) die Milchproteinfraktion stammt von Milch ab,
(2) die Milchproteinfraktion enthält Proteine mit einer Molekülmasse von 80.000 bis 85.000 Dalton, ermittelt durch Natriumdodecylsulfat-Polyacrylamidgelelektrophorese (SDS-PAGE),
(3) die Milchproteinfraktion enthält 16 bis 18 Gew.-% an basischen Aminosäuren in der konstituierenden Aminosäurezusammensetzung und hat ein Verhältnis zwischen basischer Aminosäure und saurer Aminosäure von 0,65 bis 0,85 und
(4) die Milchproteinfraktion hat einen Osteoklastogenese-Inhibitionseffekt.

2. Milchproteinfraktionsabbauprodukt, das durch den Abbau der Milchproteinfraktion nach Anspruch 1 mit einer Protease erhalten wird.

3. Osteoklastogenese-Inhibitionsmittel, das die Milchproteinfraktion nach Anspruch 1 oder das Milchproteinfraktionsabbauprodukt nach Anspruch 2 umfasst.

4. Pharmazeutikum zur Verwendung bei der Osteoklastogenese-Inhibition, das die Milchproteinfraktion nach Anspruch 1 oder das Milchproteinfraktionsabbauprodukt nach Anspruch 2 umfasst.

5. Nahrungsmittel oder Getränk zur Verwendung bei der Osteoklastogenese-Inhibition, das die Milchproteinfraktion nach Anspruch 1 oder das Milchproteinfraktionsabbauprodukt nach Anspruch 2 umfasst.

6. Futter zur Verwendung bei der Osteoklastogenese-Inhibition, das die Milchproteinfraktion nach Anspruch 1 oder das Milchproteinfraktionsabbauprodukt nach Anspruch 2 umfasst.

## Revendications

1. Fraction protéique du lait ayant les caractéristiques suivantes (1 ) à (4) :
(1) la fraction protéique du lait est dérivée du lait,
(2) la fraction protéique du lait contient des protéines ayant un poids moléculaire de 80 000 à 85 000 daltons déterminé par électrophorèse sur gel de polyacrylamide en présence de dodécylsulfate de sodium (SD-PAGE),
(3) la fraction protéique du lait contient de 16 à 18% en poids d'acides aminés de base dans la composition d'acides aminés constituante, et a un rapport des acides aminés de base sur les acides aminés acides de 0,65 à 0,85, et
(4) la fraction protéique du lait a un effet inhibiteur de l'ostéoclastogénèse.

2. Produit de dégradation de fraction protéique du lait obtenu en dégradant la fraction protéique du lait selon la revendication 1 avec une protéase.

3. Agent inhibiteur de l'ostéoclastogénèse comprenant la fraction protéique du lait selon la revendication 1 ou le produit de dégradation de fraction protéique du lait selon la revendication 2.

4. Produit pharmaceutique destiné à être utilisé pour inhiber l'ostéoclastogénèse comprenant la fraction protéique du lait selon la revendication 1 ou le produit de dégradation de fraction protéique du lait selon la revendication 2.

5. Aliment ou boisson destiné à être utilisé pour inhiber l'ostéoclastogénèse comprenant la fraction protéique du lait selon la revendication 1 ou le produit de dégradation de fraction protéique du lait selon la revendication 2.

6. Nourriture destinée à être utilisée pour inhiber l'ostéoclastogénèse comprenant la fraction protéique du lait selon la revendication 1 ou le produit de dégradation de fraction protéique du lait selon la revendication 2.
